# EUROPEAN PATENT SPECIFICATION

(11) **EP 4 346 941 B1**
(45) Date of publication and mention of the grant of the patent: **18.11.2026**
(21) Application number: 22812048.1
(22) Date of filing: 25.05.2022
(51) Int. Cl.: A61M 1/16

(54) **DEGASSING UNIT**
ENTGASUNGSEINHEIT
UNITÉ DE DÉGAZAGE

(30) Priority: 28.05.2021 US 202163194834 P
(43) Date of publication of application: 10.04.2024
(73) Proprietor: Diality Inc., Irvine CA 92618 (US)
(72) Inventor: BORILLO, Brandon, Irvine, CA 92618 (US)
(74) Representative: Dolleymores
(86) International application number: PCT/US2022/030876
(87) International publication number: WO 2022/251325

(56) References cited:
- WO-A1-2020/222654
- US-A1- 2011 092 875
- US-A1- 2014 090 557
- US-A1- 2014 157 983
- US-A1- 2014 217 028
- US-A1- 2016 166 751
- US-A1- 2018 243 494

## Description

### CROSS-REFERENCE TO RELATED APPLICATION

This application claims priority to, and the benefit of, U.S. Provisional Application No. 63/194,834, filed May 28, 2021.

### BACKGROUND OF THE INVENTION

The present invention relates to cartridges that are useful in dialysis. In particular, the present invention relates in general to the degassing of regenerated dialysate fluids.

More than 1 in 7 adults in the U.S. (approximately 15% of U.S. adults) are estimated to have chronic kidney (or renal) disease. In people who suffer from chronic kidney disease, their kidneys no longer clean their blood as well as healthy kidneys. Thus, toxic waste and extra fluid accumulates in the body. Dialysis is a treatment that removes the waste products and excess fluid that accumulate in the blood as a result of kidney failure. Chronic renal failure is when the renal function has deteriorated to about 25% of normal. This amount of deterioration causes significant changes in the blood chemistry and is about the time that people feel poorly enough that they seek medical care. If medical treatment is sought at that time, progression can be slowed. Late-stage chronic renal failure is when kidney function has decreased to 15%. End stage renal failure is when kidney function is at 5% of normal. Death will most likely result without treatment at this point.

Although there is no current cure for renal disease, there are several forms of treatment. One treatment is transplantation, which is where a human kidney is surgically placed in the body and connected to the bladder. After transplantation, daily medication is needed to keep the body from rejecting the transplanted kidney. Another treatment is peritoneal dialysis (PD). With this treatment, a mild saltwater solution containing dextrose and electrolytes called dialysate is put into the peritoneal cavity. Because there is a rich blood supply to this abdominal cavity, urea and other toxins from the blood and fluid are moved into the dialysate, thereby cleaning the blood. The dialysate is then drained from the peritoneum. Later "fresh" dialysate is again put into the peritoneum.

Another form of treatment is hemodialysis. This is a method of blood purification in which blood is continually removed from the body and passed through a dialyzer (artificial kidney) where metabolic waste and excess water are removed and pH and acid/base balance are normalized. The blood is simultaneously returned to the body. The dialyzer is a small disposable device consisting of a semi-permeable membrane. The membrane allows the wastes, electrolytes, and water to cross but restricts the passage of large molecular weight proteins and blood cells. Blood is pumped across one side of the membrane as dialysate is pumped in the opposite direction across the other side of the membrane. The dialysate is highly purified water with salts and electrolytes added. The machine is a control unit that acts to pump and control pressures, temperatures, and electrolyte concentrations of the blood and the dialysate. The average length of one hemodialysis treatment is about 3.5 hours.

There are several types of hemodialysis - including single pass systems and sorbent systems. Single pass hemodialysis is the most common treatment for renal disease. These instruments are called single pass because the dialysate (cleaning solution) passes by the blood in the dialyzer one time and then is disposed. Single pass dialysis machines generally require: (1) a water source capable of delivering at least 1000-1500 ml/min (assuming a 50% rejection rate by the reverse osmosis(R.O.) system); (2) a water purification system sufficient of providing a continuous flow of 500-800 ml/min of purified water, (3) an electrical circuit of at least 15 amps in order to pump and heal 500-800 ml of water/min, and (4) a floor drain or any other receptacle capable of accommodating at least 500 ml of used dialysate/minute as well as the rejected water from the R.O. system.

Sorbent dialysis systems do not require a continuous water source, a separate water purification machine, or a floor drain because the system continuously regenerates a small volume of dialysate and incorporates a water treatment system within the machine. Therefore, sorbent systems are portable. The sorbent system can use 6 liters of water from which dialysate is made for an entire treatment. The sorbent system uses a sorbent cartridge, which acts both as a water purifier and as a means to regenerate used dialysate into fresh dialysate. The infusate system acts with the sorbent system to properly balance the electrolyte composition of the regenerated dialysate.

Currently, there are several types of multi-layer sorbent cartridges used in dialysis machines that remove uremic toxins from patient's blood and reuse the dialysate solution via recirculation. These sorbent dialysis systems, however, do not efficiently remove uremic toxins at a high capacity or with a high electrolyte stability.

In a sorbent dialysis system, the process of removing urea with urease and zirconium phosphate generates a fluid composition leaving the sorbent cartridge that has a lower pH than the standard range used in bicarbonate dialysate. Previous sorbent devices have used high pH final layers in sorbent cartridges of zirconium oxide and zirconium carbonate. These devices, however, have limitations on dialysate flow rate and dialysis treatment length that must be traded off against increasing the total mass of the final layers in order to raise the pH. Alternatively, an increased pH can be used in the zirconium phosphate layer(s). Other metals phosphates of iron (III), titanium (IV) and cerium (IV) suffer from similar issues. This, however, results in excessive sodium and reduced removal capacity for the ammonium generated by the urea reaction with urease. Other processes have used sodium bicarbonate as an added chemical to the sorbent outlet stream, but these systems either have resultant pH levels well below standard bicarbonate dialysis or require specialized systems for the removal of a majority of the dissolved CO₂ gas in the fluid stream leaving the sorbent cartridge.

Some devices removed CO₂ by pumping dialysate into an open chamber and allowing bubbles to float to the top of the chamber. This system, however, only relies on gas naturally exiting the fluid while agitating the fluid with a pump. Such a system affords less control over the degassing process. Moreover, the fluid reservoir is open to the atmosphere, which presents potential sterility issues, extra short-term maintenance, and long-term maintenance issues.

Other degassing systems that remove CO₂ under vacuum are not ideal because they require a lot of energy to operate. Moreover, a lot of water evaporates under these vacuum systems, which concentrates the dialysate causing sodium levels to rise. They also create a stream of moisture that needs to be collected and disposed. A vacuum system also adds larger vacuum pressure requirements on system tubing and pumps. Membrane degassing systems require large surface area and expensive membranes that are prone to fouling issues. Degassing with a chemical reaction or with an adsorbent adds considerable cost and most feasible sorbents for CO₂ also leach undesired chemicals.

Relevant prior art is for instance disclosed in document US2018/243494 A1.

Accordingly, in the area of dialysis, it would be beneficial to create a sorbent regeneration system that includes an effective method of removing excess CO₂ gas from the regenerated fluid while also raising the pH of the fluid to an acceptable level.

### BRIEF DESCRIPTION OF THE DRAWINGS

FIG. 1A is an exemplary diagram of a sorbent cartridge with a degasser module.
FIG. 1B is a cross-section of an exemplary sorbent cartridge with a degasser module.
FIG. 2A is a bottom side perspective view of a portion of a degasser module.
FIG. 2B is a top side perspective view of a portion of a degasser module.
FIG. 3A is a perspective view of a degasser module.
FIG. 3B is a cross-section A-A' of the degasser module shown in FIG. 3A.
FIG. 3C is a cross-section B-B' of the degasser module shown in FIG. 3A.

### SUMMARY

A degassing module according to the present invention comprises the technical features as defined in independent claim 1. A system for dialysis according to the present invention comprises the technical features as defined in independent claim 7. A method of degassing a regenerated dialysate comprising CO2 according to the present invention comprises the technical features as defined in independent claim 10.

A sorbent cartridge, which includes a degassing module, is described that can be used to regenerate dialysate to be used in a dialysis system.

Sorbent regeneration degassing may be accomplished by introducing a stream of air, which may be substantially free of CO₂, into the regenerated dialysate. The stream of air may have about the same approximate flowrate as the flowrate of the liquid. The combined air-liquid fluid may then be exposed to a hydrophobic membrane where the gas is free to exit the system.

The fluid may be directed to flow through several fluid channels. In a first fluid channel, the fluid may be mixed with ambient air to lower the partial pressure of CO₂ in the fluid. In a second fluid channel, as the fluid flows through the long channel, at least a portion of the gas contained in the fluid dissipates out of the fluid through a hydrophobic membrane.

### DETAILED DESCRIPTION

In a dialysis treatment, a dialysis machine functions as an artificial kidney to remove uremic toxins, balance ionic molecules, and equilibrate pH for patients that lack healthy kidneys to perform those tasks. With the substitution of a multi-layer cartridge for a large volume of water, the cartridge is responsible for not only removing uremic toxins, but also balancing ionic salts and pH.

A hemodialysis system that incorporates a dialysate generator is described in US 2021/0128807. Various embodiments of a sorbent regeneration system, which includes a sorbent cartridge, described herein may be incorporated into the system described in US 2021/0128807 in place of the dialysate generator.

A sorbent regeneration cartridge 200 may include multiple layers of components to clean the dialysate. The cartridge 200 may at least include a layer of urease and a layer or layers of zirconium phosphate, or other metal phosphates such as iron (III), titanium (IV), and cerium(IV), which may be in different forms. During regenerative dialysis, the used dialysate moves up through the layers of the cartridge 200. The urease converts urea into ammonium carbonate. The ammonia and ammonium ions are then removed by the zirconium phosphate (or metal phosphate) layer or layers in exchange for H⁺ and Na⁺ ions. The carbonate from the urea hydrolysis then combines with H⁺ to form bicarbonate (HCO₃⁻) and carbonic acid (H₂CO₃). Carbonic acid is an unstable organic acid; most of it quickly breaks down into water and carbon dioxide molecules (CO₂). The CO₂ gas bubbles may then be vented from the cartridge using degassing unit 300.

The sorbent cartridge 200 has two processes that create an excess amount of carbon dioxide (CO₂) gas. The breakdown of urea (CON₂H₄) by the urease enzyme follows the reactions below:

CON₂H₄ + H₂O → CO₂ + 2NH₃

CO₂ + 2NH₃ H₂O → 0.5CO₃²⁻ HCO₃¹⁻ + 2NH₄¹⁺

The chemical equilibrium may have different amounts of each form of carbon, ammonia (NH₃), and ammonium (NH₄⁺) depending on the solution pH:

xCO₂ ←→ yHCO₃¹⁻ ←→zCO₃²⁻

aNH₃ ←→ bNH₄¹⁺

When the spent dialysate, which contains ammonia (NH₃), then passes through the zirconium phosphate (or other metal phosphate) cation adsorbent, the following reaction occurs, which results in the production of carbonic acid (H₂CO₃).

Zr(HPO₄)₂ + 0.5CO₃²⁻ HCO₃¹⁻ + 2NH₄¹⁺ ←→ Zr(NH₄PO₄)₂ + HCO₃¹⁻ + H₂CO₃

To a lesser extent sodium bicarbonate and sodium chloride can also exchange to produce carbonic acid.

Zr(HPO₄)₂ + NaHCO₃ + NaCl ←→ Zr(NaPO₄)(HPO₄) + H₂CO₃ + NaCl

Carbonic acid (H₂CO₃) is not stable and tends to breakdown into carbon dioxide (CO₂), according to the following equation:

H₂CO₃ → CO₂ + H₂O

Thus, treatment of the dialysate with the zirconium phosphate or other metal phosphates such as iron (III), titanium (IV) and cerium (IV) results in an acidified dialysate solution. A portion of the CO₂ present may be in a purely gaseous phase and another portion of the CO₂ present may be dissolved in the dialysate exiting the sorbent cartridge 200. The portion of CO₂ dissolved in the dialysate may be very high because the partial pressure of CO₂ in the sorbent cartridge is very high. This relationship is known as Henry's law: ${H}^{cp} = \frac{{C}_{i}}{{P}_{i}}$

In Henry's Law, a constant (*H^{cp}*) relates the concentration of a dissolved gas (*Cᵢ*) to the partial pressure of that same dissolved gas (*Pᵢ*). Thus, methods to remove excess CO₂ may focus on lowering the partial pressure of CO₂ in the dialysate.

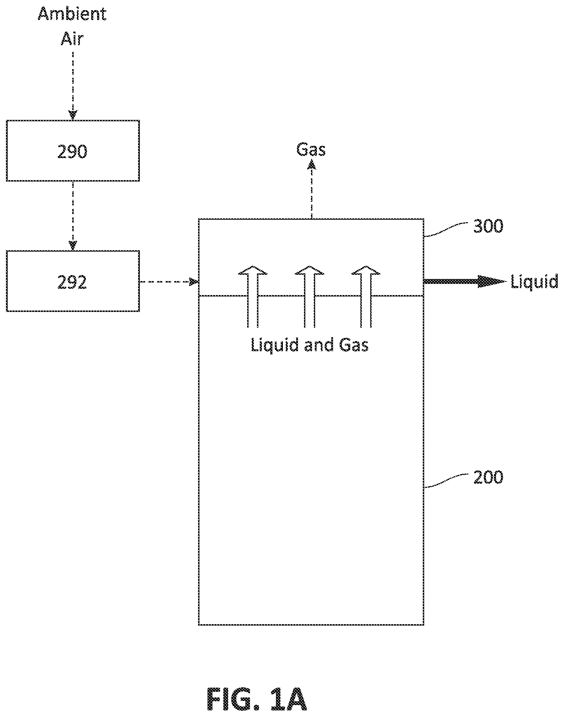

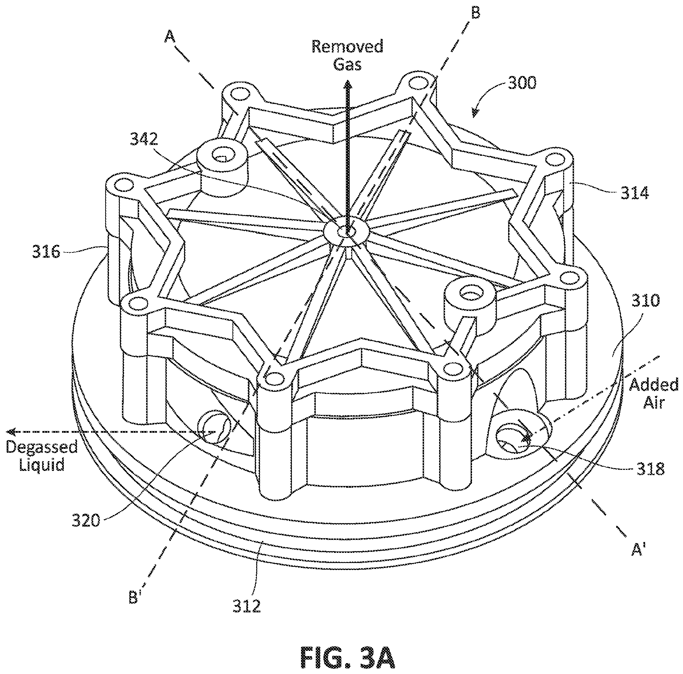

As seen in FIGS. 1A-1B and FIG. 3A, a degasser module or unit 300 may be located on top of a sorbent cartridge 200. The degasser 300 may include a container 316 having a base 310 and a cover 314. The base may have a groove 312 configured to hold an O-ring 322, which can frictionally engage an outer wall 296 of the sorbent cartridge 200. Container 316 may contain at least two fluid channels, a lower fluid channel 330 and an upper fluid channel 340, which are configured to direct flow of a fluid, such as regenerated dialysate. The base 310 of the degasser unit 300 may sit adjacent to or in contact with the particle retaining filter 298 that sits atop the sorbent bed 294 such that the lower fluid channel may be in contact the particle retaining filter 298 at the top of the sorbent bed 294. Thus, the lower fluid channel 330 may be in contact with the fluid and gas that is being pumped from the bottom to the top of the sorbent bed 294 of sorbent cartridge 200.

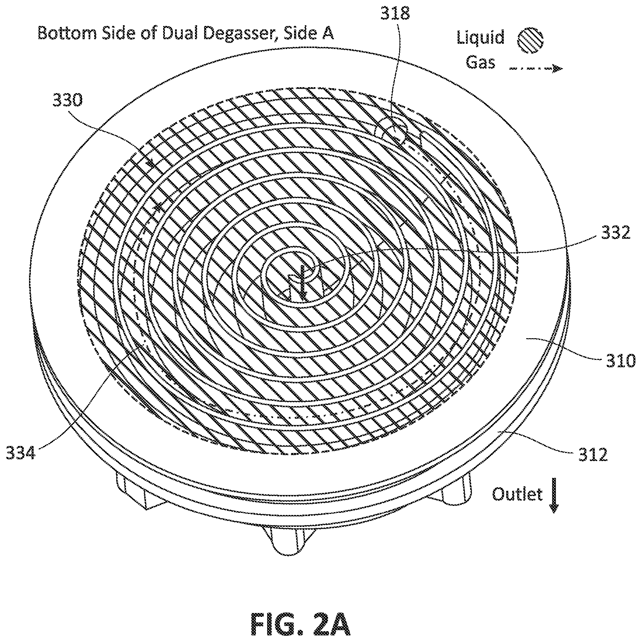

As seen in FIG. 1A, pump 290 may pump a stream of ambient air through a microbial membrane filter 292 through an inlet 318. As seen in FIG. 2A, inside the container 316, the airstream 334 may be combined with the fluid pumped from the sorbent bed 294 into the lower fluid channel 330. The fluid may enter the lower fluid channel 330 anywhere along the pathway of the lower fluid channel 330. The addition of the airstream into the lower fluid channel 330 may cause the fluid and gas from the sorbent cartridge to follow the path defined by the lower fluid channel 330, leading to a port 332 that fluidly connects the lower fluid channel 330 and the upper fluid channel 340.

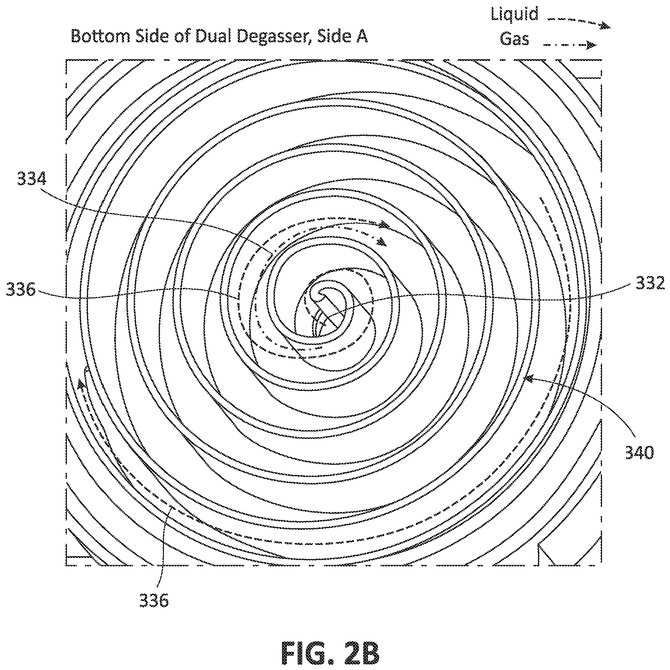

Each of the lower fluid channel 330 and the upper fluid channels may be a long, narrow channel. Each fluid channel may be a length of at least about 6 inches (1 inch = 2,54 cm), alternatively at least about 7 inches, alternatively at least about 8 inches, alternatively at least about 9 inches, alternatively at least about 10 inches, alternatively between about 6 inches and about 12 inches, alternatively between about 7 inches and about 11 inches, alternatively between about 8 inches and about 11 inches, alternatively between about 6 inches and about 25 inches, alternatively between about 6 inches and about 20 inches, alternatively between about 6 inches and about 15 inches in length. Each of the lower fluid channel 330 and the upper fluid channels may be in a spiral pattern (*see, e.g.,* FIGS. 2A and 2B), a switchback pattern, random, or other suitable arrangement that first the length of the fluid path within the container 316. In embodiments where the patterns of the lower fluid channel 330 and the upper fluid channel 340 are spiral, the spiral of each may be either clockwise or counterclockwise from the center (e.g., as viewed from the top of the degassing unit 300). In some embodiments, the spirals of each may be in the same clockwise or counterclockwise direction or the spirals of each may be in different directions such that one is clockwise, and the other is counterclockwise.

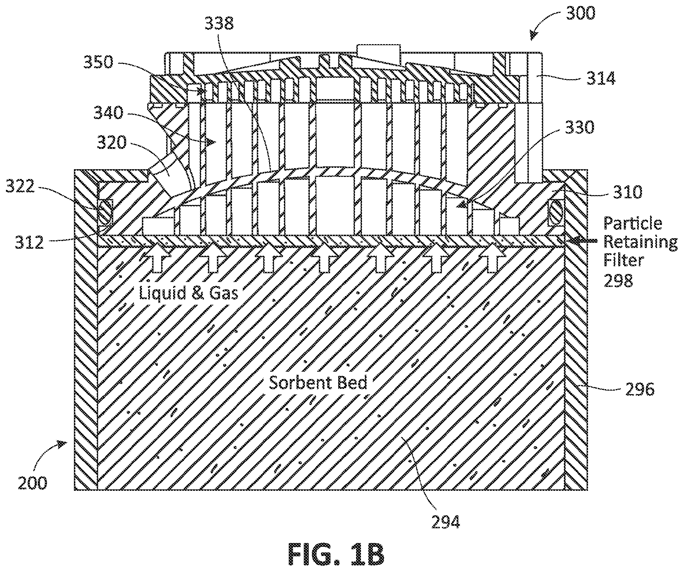

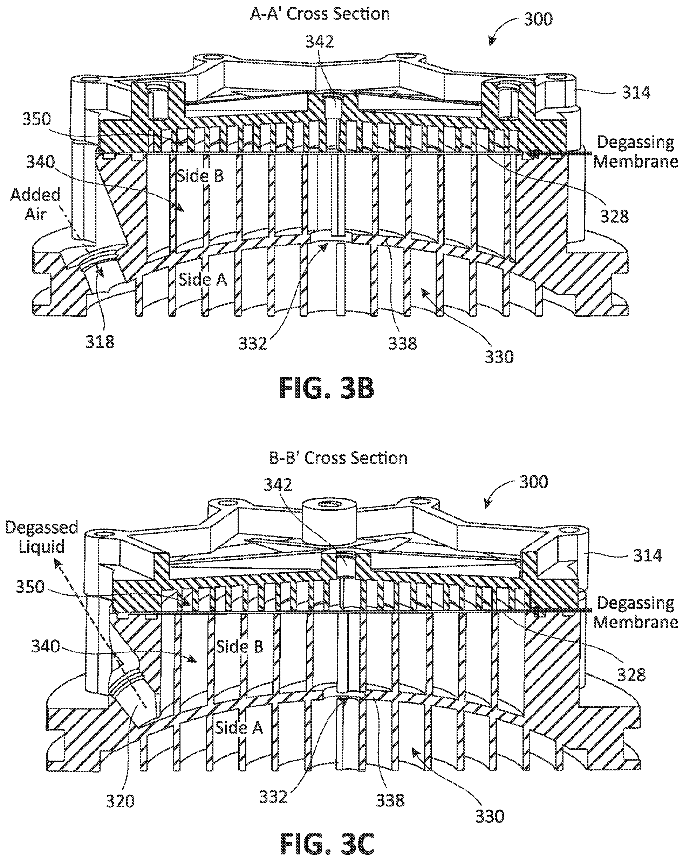

As seen in FIGS. 1B, 3B, and 3C, a surface 338 separates the lower fluid channel 330 and the upper fluid channel 340 such that the lower 330 and upper 340 fluid channels are only in fluid communication through port 332. The surface 338 may have a dome (see, e.g., FIGS. 3B and 3C) or conical shape, such that the port 332 is at a greater height or elevation than an outer edge of the lower fluid channel 330. In some embodiments, the port may be at least about 0.125 inches, alternatively at least about 0.25 inches, alternatively at least about 0.4 inches, alternatively at least about 0.5 inches, alternatively between about 0.125 inches to about 0.8 inches, alternatively between about 0.125 inches to about 0.7 inches, alternatively between about 0.125 inches to about 0.6 inches, alternatively between about 0.125 inches to about 0.5 inches higher than an outer edge of the lower fluid channel 330. In embodiments where the surface has a dome shape, the port 332 may be located at or near the center of the dome. Thus, the surface 338 defining the top of the lower fluid channel 330 may have a concave surface and the surface 338 defining the bottom of the upper fluid channel 340 may have a convex surface. Alternatively, the surface 338 may be level, horizontal (not shown). In embodiments where the lower fluid channel 330 and the upper fluid channel 340 each have a spiral shape or pattern, the port 332 connecting the channels may be located at the center of each of the spirals and the air inlet port 318 and liquid outlet port 320 may be located at or near an outer edge of the lower fluid channel 330 and the upper fluid channel 340, respectively.

As seen in FIGS. 1A, 1B, and 2A, the air stream may be pumped through air inlet 318 into the lower fluid channel 330, where the air stream 334 may mix with the fluid, e.g., regenerated dialysate, in the lower fluid channel 330 that is being pumped up from the sorbent bed 294. The fluid may enter the lower fluid channel 330 anywhere along the pathway of the lower fluid channel 330. The flow rate of the ambient air stream into the degassing unit 300 may be between about 100 mL/min and about 600 mL/min, alternatively between about 200 mL/min and about 600 mL/min, alternatively between about 200 mL/min and about 500 mL/min, alternatively between about 200 mL/min and about 400 mL/min. The flow rate of the fluid and gas from the sorbent bed 294 may be between about 100 mL/min and about 600 mL/min, alternatively between about 200 mL/min and about 600 mL/min, alternatively between about 200 mL/min and about 500 mL/min, alternatively between about 200 mL/min and about 400 mL/min. In some embodiments, the flow rate of the ambient air and the flow rate of the fluid and gas from the sorbent bed 294 are substantially the same. Alternatively, the difference between the flow rate of the ambient air and the flow rate of the fluid may be between about 50 mL/min to about 100 mL/min, alternatively between about 50 mL/min to about 200 mL/min, alternatively between about 100 mL/min to about 300 mL/min, alternatively between about 100 mL/min to about 400 mL/min, alternatively between about 200 mL/min to about 400 mL/min, alternatively between about 300 mL/min to about 500 mL/min.

As seen in FIG. 2A, the path of the air stream 334 may force the fluid to flow along through the path defined by the lower fluid channel 330 until it reaches and flows through the port 332 to the upper fluid channel. The long path defined by the lower fluid channel 330 enables the mixing of the air stream 334 from the ambient air and the fluid, which contains CO₂. As the fluid flows through the lower fluid channel 330, the CO₂ partial pressure in the fluid (e.g., regenerated dialysate) may equilibrate with the ambient air, thereby lowering the total amount of dissolved CO₂. As explained above with reference to Henry's law, lowering the partial pressure of CO₂ for the liquid lowers the total amount of dissolved CO₂.

After exiting the lower fluid channel 330 through the port 332 into the upper fluid channel 340 through port 332, the fluid flows through the path 336 defined by the upper fluid channel 340 until it reaches liquid outlet port 320, which may be located at or near an outer edge of the upper fluid channel 340. A flow rate of the fluid at or near the center of the upper fluid channel 340 may be higher than a flow rate of the fluid at or near an outer edge of the upper fluid channel 340 due to a higher pressure near the center of the upper fluid channel 340. The long pathway defined by the upper fluid channel 340 allows at least a portion of the gas contained in the fluid to dissipate out of the fluid and into the atmosphere as the fluid follows path 336 through the upper fluid channel 340. The long length of the upper fluid channel 340 may give the gas and liquid time to separate. As the fluid containing the gas moves around the upper fluid channel (e.g., path 336 of the spiral channel shown in FIG. 2B), the fluid containing the gas may experience centripetal forces, which are greater on the liquid because of its greater mass as compared to a mass of the gas. This greater force required for changing the direction of the liquid may result in the liquid being forced against the outside of the upper fluid channel 340 (e.g., outer walls of the spiral depicted in FIG. 2B). This may help to collate small gas bubbles at the center of the upper fluid channel 340 or at the inner walls of the upper fluid channel 340, thereby allowing them to rise to a hydrophobic degassing membrane 328 because larger bubbles are less likely to remain mixed in the liquid stream or stick to surfaces. Thus, the centripetal force may help to separate the gas (e.g., CO₂) from the liquid. As the gas separates from the liquid in upper fluid channel 340, the gas may pass through the hydrophobic membrane 328 located above the upper fluid channel 340, while the liquid may exit out of the liquid outlet 320 at the bottom of the channel.

The hydrophobic degassing membrane 328 may be located on top of the upper fluid channel 340 and may be in contact with the fluid flowing through the upper fluid channel 340. Because it is hydrophobic, in general, fluid will not pass through the membrane 328 (i.e., it is impermeable to liquid), but will allow gas to pass through the membrane. The fluid contacting the hydrophobic membrane 328 may be under a modest amount of back pressure (e.g., <5 psi) allowing CO₂ and air bubbles to leave the fluid stream. The hydrophobic degassing membrane 328 may have a pore size between about 0.005 µm to about 1.5 µm, alternatively between about 0.005 µm to about 1.3 µm, alternatively between about 0.005 µm to about 1.0 µm, alternatively between about 0.006 µm to about 1.0 µm.

As seen in FIG. 3A, a cover 314 over the container 316 may be added to close the system and reduce environmental contaminants from entering the fluid. The cover 314 may contain an opening 342 to allow the gas (e.g., the CO₂ from the fluid) to escape into the atmosphere. As seen in FIGS. 1B, 3B, and 3C, the cover 314 may also contain an additional structure 350 to assist in keeping the hydrophobic membrane 328 substantially flat. Without the additional structure 350, the hydrophobic membrane 328 may form a curved, concave surface with a higher point in a center of the hydrophobic membrane 328 as compared to an outer edge of the hydrophobic membrane 328 due to pressure in the container 316. If the hydrophobic membrane 328 was no longer substantially flat, a portion of the hydrophobic membrane 328 may not be in contact with the liquid in the upper fluid channel 340. The additional structure 350 may be in any form that assists in keeping the hydrophobic membrane 328 substantially flat or planar, along with keeping some void space above the membrane to allow the gas to escape through the opening 342. The additional structure 350 may depend on the pattern of the upper fluid channel 340 as the additional structure 350 may complement the pattern of the upper fluid channel 340. For example, in embodiments where the upper fluid channel 340 is in a spiral configuration, the additional structure 350 may also be in a complementary spiral configuration, where at least a portion of the walls defining the spiral in the additional structure 350 may be positioned between the walls defining the spiral configuration of upper fluid channel 340, as seen in FIGS. 3B and 3C.

As used herein and in the appended claims, the singular forms "a," "an," and "the" include plural referents unless the context clearly dictates otherwise.

While specific examples have been shown in the drawings and are herein described in detail, it should be understood, however, that these embodiments are not to be limited to the particular form disclosed and that the invention is defined by the appended claims.

## Claims

1. A degassing module 300 configured for use in a system for dialysis that comprises a sorbent regeneration cartridge 200 comprising urease and metal phosphate, wherein the degassing module is configured for degassing dialysate fluid that has flown through the sorbent regeneration cartridge the degassing module comprising:
an air inlet port (318);
a dialysate outlet port (320);
first (330) and second (340) channels located in an interior chamber, wherein each of the first and second channels are configured to direct a flow of a fluid, wherein the first channel (330) is a lower channel configured for dialysate fluid to enter anywhere along the pathway of the lower channel and wherein the first channel is in communication with the air inlet port and the second channel (340) is in communication with the dialysate outlet port;
a port (332) connecting the first and second channels;
a hydrophobic membrane (328) positioned above the second channel; and
a cover (314) with a gas outlet port (342); wherein the cover is positioned above the hydrophobic membrane.

2. The module of claim 1, wherein the first channel and the second channel each have a spiral configuration.

3. The module of claim 1, wherein the port connecting the first and the second channels is located at or near a center of first channel and at or near a center of the second channel.

4. The module of claim 1, wherein the first channel has a spiral configuration having a center of the spiral configuration and an outer edge, and wherein the air inlet port is located at the outer edge.

5. The module of claim 1, further comprising a substrate (338) separating the first and second channels, wherein the substrate comprises the port connecting the first and second channels.

6. The module of claim 5 wherein the substrate comprises a dome shape, wherein the port is located approximately near a center of the dome shape.

7. A system for dialysis, comprising:
a sorbent regeneration cartridge (200) comprising urease and metal phosphate, and
a degassing module (300) as claimed in any preceding claim.

8. The system of claim 7, wherein the metal phosphate is zirconium phosphate, or is selected from the group consisting of iron (III) phosphate, titanium (IV) phosphate, cerium (IV) phosphate, and combinations thereof, or

9. The system of claim 7, wherein the metal phosphate comprises a mixture of at least two metal phosphates, or wherein the metal phosphate comprises a mixture of zirconium phosphate and titanium phosphate.

10. A method of degassing a regenerated dialysate comprising CO₂, **characterized by** the method comprising the steps of:
pumping a flow of air into a stream of regenerated dialysate into a first channel (330) to form an aerated fluid stream,
wherein the aerated fluid stream flows through the first channel, exits a port (332) and flows through a second channel (340), and wherein bubbles of CO₂ are released from the aerated fluid in the second channel.

11. The method of claim 10, wherein the flow of air has a flow rate of between about 300 mL/min and about 500 mL/min.

12. The method of claim 10, further comprising the step of pumping the stream of regenerated dialysate into the first channel, wherein the stream of regenerated dialysate is pumped at a flow rate of between about 300 mL/min and about 500 mL/min, optionally
wherein the flow rate of the stream of regenerated dialysate is about the same as a flow rate of the flow of air into the first channel.

13. The method of claim 10, wherein the bubbles of CO₂ are released through a hydrophobic membrane (328) located on top of the second channel, or
the second channel has a spiral shape, and optionally
wherein the port is located at about a center of the spiral shape of the first channel and at about a center of the spiral shape of the second channel, or
wherein the second channel is positioned on top of the first channel.

## Patentansprüche

1. Entgasungsmodul (300), das für die Verwendung in einem Dialysesystem konfiguriert ist, das eine Sorptionsmittelregenerationskartusche (200) umfasst, die Urease und Metallphosphat umfasst, wobei das Entgasungsmodul dazu konfiguriert ist, Dialysatflüssigkeit zu entgasen, die durch die Sorptionsmittelregenerationskartusche geflossen ist, wobei das Entgasungsmodul umfasst:
einen Lufteinlassanschluss (318);
einen Dialysatauslassanschluss (320);
einen ersten (330) und einen zweiten (340) Kanal, die sich in einer Innenkammer befinden, wobei jeder des ersten und des zweiten Kanals dazu konfiguriert sind, einen Flüssigkeitsfluss leiten, wobei der erste Kanal (330) ein unterer Kanal ist, der derart konfiguriert ist, dass Dialysatflüssigkeit entlang des gesamten Wegs des unteren Kanals eintritt, und wobei der erste Kanal mit dem Lufteinlassanschluss in Verbindung steht und der zweite Kanal (340) mit dem Dialysatauslassanschluss in Verbindung steht;
ein Anschluss (332), die den ersten und den zweiten Kanal verbindet;
eine hydrophobe Membran (328), die über dem zweiten Kanal positioniert ist; und
eine Abdeckung (314) mit einem Gasauslassanschluss (342);
wobei die Abdeckung über der hydrophoben Membran angeordnet ist.

2. Modul nach Anspruch 1, wobei der erste Kanal und der zweite Kanal jeweils eine spiralförmige Konfiguration aufweisen.

3. Modul nach Anspruch 1, wobei sich die den ersten und den zweiten Kanal verbindende Öffnung an oder nahe der Mitte des ersten Kanals und an oder nahe der Mitte des zweiten Kanals befindet.

4. Modul nach Anspruch 1, wobei der erste Kanal eine spiralförmige Konfiguration mit einer Mitte der spiralförmigen Konfiguration und einem Außenrand aufweist und wobei sich der Lufteinlassanschluss an dem Außenrand befindet.

5. Modul nach Anspruch 1, ferner umfassend ein Substrat (338), das den ersten und den zweiten Kanal trennt, wobei das Substrat den Anschluss umfasst, der den ersten und den zweiten Kanal verbindet.

6. Modul nach Anspruch 5, wobei das Substrat eine Kuppelform aufweist, wobei sich der Anschluss ungefähr nahe einer Mitte der Kuppelform befindet.

7. System für die Dialyse, das umfasst:
eine Sorptionsmittelregenerationskartusche (200), die Urease und Metallphosphat umfasst, und
ein Entgasungsmodul (300) nach einem der vorstehenden Ansprüche.

8. System nach Anspruch 7, wobei das Metallphosphat Zirkoniumphosphat ist oder aus der Gruppe ausgewählt ist, bestehend aus Eisen(III)-phosphat, Titan(IV)-phosphat, Cer(IV)-phosphat und Kombinationen davon, oder

9. System nach Anspruch 7, wobei das Metallphosphat eine Mischung aus wenigstens zwei Metallphosphaten umfasst oder wobei das Metallphosphat eine Mischung aus Zirkoniumphosphat und Titanphosphat umfasst.

10. Verfahren zum Entgasen eines regenerierten Dialysats, das CO₂ umfasst, **gekennzeichnet dadurch, dass** das Verfahren die Schritte umfasst:
Pumpen eines Luftstroms in einen Fluss regenerierten Dialysats in einen ersten Kanal (330), um einen belüfteten Flüssigkeitsfluss auszubilden,
wobei der belüftete Flüssigkeitsfluss durch den ersten Kanal fließt, aus einem Anschluss (332) austritt und durch einen zweiten Kanal (340) fließt, und wobei CO₂-Blasen aus der belüfteten Flüssigkeit in dem zweiten Kanal freigesetzt werden.

11. Verfahren nach Anspruch 10, wobei der Luftstrom eine Durchflussrate zwischen etwa 300 ml/min und etwa 500 ml/min aufweist.

12. Verfahren nach Anspruch 10, das ferner den Schritt des Pumpens des Flusses von regeneriertem Dialysat in den ersten Kanal umfasst, wobei der Fluss von regeneriertem Dialysat mit einer Durchflussrate zwischen etwa 300 ml/min und etwa 500 ml/min gepumpt wird, optional
wobei die Durchflussrate des Flusses von regeneriertem Dialysat in etwa der Durchflussrate des Luftstroms in den ersten Kanal entspricht.

13. Verfahren nach Anspruch 10, wobei die CO₂-Blasen durch eine hydrophobe Membran (328) freigegeben werden, die sich oberhalb des zweiten Kanals befindet, oder
der zweite Kanal eine spiralförmige Form aufweist und optional
wobei sich der Anschluss in etwa in der Mitte der spiralförmigen Form des ersten Kanals und in etwa in der Mitte der spiralförmigen Form des zweiten Kanals befindet, oder
wobei der zweite Kanal oberhalb des ersten Kanals positioniert ist.

## Revendications

1. Module de dégazage (300) configuré pour utilisation dans un système de dialyse qui comprend :
une cartouche de régénération de sorbant (200) comprenant de l'uréase et du phosphate métallique, où le module de dégazage est configuré pour dégazer un fluide de dialysat qui a coulé à travers la cartouche de régénération de sorbant
le module de dégazage comprenant :
un orifice d'entrée d'air (318),
un orifice de sortie de dialysat (320),
un premier (330) et un deuxième (340) canal situés dans une chambre intérieure, dans lequel chacun du premier et du deuxième canal est configuré pour diriger un écoulement de fluide, dans lequel le premier canal (330) est un canal inférieur configuré pour que le fluide de dialysat entre n'importe où le long du chemin du canal inférieur et dans lequel le premier canal est en communication avec l'orifice d'entrée d'air et le deuxième canal (340) est en communication avec l'orifice de sortie de dialysat ;
un orifice (332) raccordant le premier et le deuxième canal ;
une membrane hydrophobe (328) positionnée au-dessus du deuxième canal ; et
un couvercle (314) doté d'un orifice de sortie de gaz (342) ;
dans lequel le couvercle est positionné au-dessus de la membrane hydrophobe.

2. Module selon la revendication 1, dans lequel le premier canal et le deuxième canal ont chacun une configuration en spirale.

3. Module selon la revendication 1, dans lequel l'orifice raccordant le premier et le deuxième canal est situé au ou près du centre du premier canal et au ou près du centre du deuxième canal.

4. Module selon la revendication 1, dans lequel le premier canal a une configuration en spirale ayant un centre de la configuration en spirale et un bord extérieur, et dans lequel l'orifice d'entrée d'air est situé au bord extérieur.

5. Module selon la revendication 1, comprenant en outre un substrat (338) séparant le premier et le deuxième canal, dans lequel le substrat comprend l'orifice raccordant le premier et le deuxième canal.

6. Module selon la revendication 5, dans lequel le substrat comprend une forme de dôme, dans lequel l'orifice est situé approximativement près du centre de la forme de dôme

7. Système de dialyse, comprenant :
une cartouche de régénération de sorbant (200) comprenant de l'uréase et du phosphate métallique, et
un module de dégazage (300) selon l'une quelconque des revendications précédentes.

8. Système selon la revendication 7, dans lequel le phosphate métallique est du phosphate de zirconium, ou est sélectionné parmi le groupe composé de phosphate de fer (III), de phosphate de titane (IV), de phosphate de cérium (IV) et de combinaisons de ceux-ci, ou

9. Système selon la revendication 7, dans lequel le phosphate métallique comprend un mélange d'au moins deux phosphates métalliques, ou dans lequel le phosphate métallique comprend un mélange de phosphate de zirconium et de phosphate de titane.

10. Procédé de dégazage d'un dialysat régénéré comprenant du CO₂, **caractérisé par** le procédé comprenant les étapes consistant à :
pomper un écoulement d'air dans un courant de dialysat régénéré dans un premier canal (330) pour former un courant de fluide aéré,
dans lequel le courant de fluide aéré coule à travers le premier canal, sort d'un orifice (332) et coule à travers un deuxième canal (340), et dans lequel des bulles de CO₂ sont libérées du fluide aéré dans le deuxième canal.

11. Procédé selon la revendication 10, dans lequel l'écoulement d'air a un débit d'entre environ 300 ml/min et environ 500 ml/min.

12. Procédé selon la revendication 10, comprenant en outre l'étape consistant à pomper le courant de dialysat régénéré dans le premier canal, dans lequel le courant de dialysat régénéré est pompé à un débit d'entre environ 300 ml/min et environ 500 ml/min, optionnellement dans lequel le débit du courant de dialysat régénéré est environ le même que le débit de l'écoulement d'air dans le premier canal.

13. Procédé selon la revendication 10, dans lequel les bulles de CO₂ sont libérées à travers une membrane hydrophobe (328) située au-dessus du deuxième canal, ou le deuxième canal a une forme en spirale, et optionnellement
dans lequel l'orifice est situé à peu près au centre de la forme en spirale du premier canal et à peu près au centre de la forme en spirale du deuxième canal, ou
dans lequel le deuxième canal est positionné au-dessus du premier canal.
